(19) Europäisches Patentamt
European Patent Office
Office européen des brevets

(11) Veröffentlichungsnummer: **0 385 287 A2**

(12) # EUROPÄISCHE PATENTANMELDUNG

(21) Anmeldenummer: **90103506.3**

(51) Int. Cl.5: **C07H 13/04**

(22) Anmeldetag: **23.02.90**

(30) Priorität: **28.02.89 DE 3906174**

(43) Veröffentlichungstag der Anmeldung: **05.09.90 Patentblatt 90/36**

(84) Benannte Vertragsstaaten: **CH DE DK LI NL**

(71) Anmelder: **Forschungszentrum Jülich GmbH**
**Postfach 1913**
**D-5170 Jülich(DE)**

(72) Erfinder: **Giannis, Athanassios, Dr.**
**Londoner Strasse 13**
**D-5300 Bonn 1(DE)**
Erfinder: **Henk, Thomas**
**Burgstrasse 54 B**
**D-5300 Bonn 2(DE)**
Erfinder: **Sandhoff, Konrad, Prof., Dr.**
**Auf dem Platt 12**
**D-5304 Alfter 4(DE)**

(54) **Verfahren zur Herstellung von D-Mannosaminderivaten.**

(57) Die Herstellung von D-N-Mannosaminderivaten aus den entsprechenden Glucosaminderivaten erfolgt erfindungsgemäß, indem zunächst das Glucosaminderivat in einer Wittig-Reaktion mit Phosphor-Ylid unter Epimerisierung zum entsprechenden olefinischen Derivat umgesetzt wird, das nach Abtrennung von der Reaktionsmischung mit Ozon zum Mannosaminderivat oxydiert wird. Die Umsetzungen erfolgen mit relativ hohen Ausbeuten, und die Abtrennung des durch Wittig-Reaktion gebildeten olefinischen Mannosaminderivats kann durch Säulenchromatographie mit Chloroform/Methanol (5:1) vom Glucosaminderivat erfolgen oder durch einfache Kristallisation, wenn von einem 4,6-O-Benzyliden-Glucosaminderivat ausgegangen wird, dessen Benzylidengruppe bei der nachfolgenden Ozonolyse durch geeignete Temperaturwahl gleichzeitig abgespalten werden kann.

EP 0 385 287 A2

## Verfahren zur Herstellung von D-Mannosaminderivaten

Die Erfindung bezieht sich auf ein Verfahren zur Herstellung von D-Mannosaminderivaten der Formel I oder II

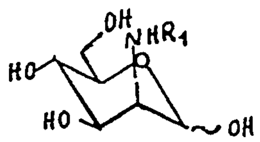

(I)

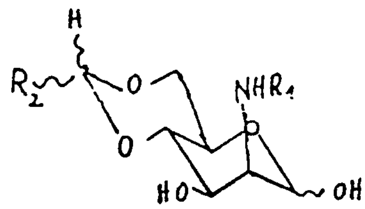

(II)

in der $R_1$ ein Acylrest und $R_2$ ein gegebenenfalls substituierter aromatischer Rest ist.

D-Mannosaminderivate der Formel I oder mit zusätzlicher Schutzgruppe gemäß Formel II sind für Synthesezwecke brauchbar, insbesondere zur Herstellung von Neuraminsäure (Man-Jo Kim u. a.; J. Am. chem. Soc. 110 (1988) 6481-86). Bisher wurde N-Acetylmannosamin durch basenkatalysierte Epimerisierung aus N-Acetylglucosamin hergestellt unter Erzielung relativ geringer Ausbeuten.

Ziel der Erfindung ist ein Verfahren zur Herstellung von D-Mannosaminderivaten, das auf relativ einfache Weise zu akzeptablen Ausbeuten führt und ohne daß aufwendige Schutzgruppeneinführungen notwendig wären.

Das zu diesem Zweck entwickelte erfindungsgemäße Verfahren ist dadurch gekennzeichnet, daß man das entsprechende D-Glucosaminderivat der Formel III oder IV

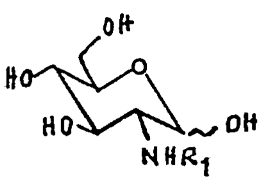

( III )

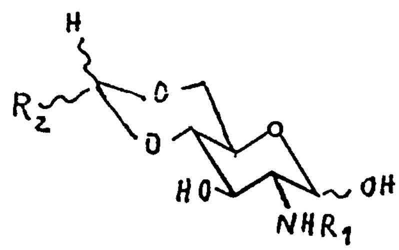

( IV )

in der $R_1$ und $R_2$ die bereits angegebene Bedeutung haben, in einer Wittig-Reaktion mit einem Phosphor-Ylid unter Epimerisierung umsetzt und das gebildete olefinische D-Mannosaminderivat nach Abtrennung des D-Glucosaminderivats mit Ozon oxydiert.

Als Phosphor-Ylid wird dabei insbesondere eine Verbindung der Formel V

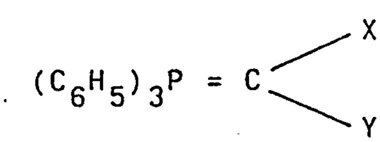

( V )

verwendet, in der X und Y gleich oder verschieden sein können und ein Wasserstoffatom, einen Phenyl-, Nitrophenyl-, Chlorphenyl-, Phenyl-$C_1$-$C_8$-alkoxy oder Furylreste bedeuten, wobei X und Y nicht gleichzeitig

Wasserstoff sein sollen.

Bevorzugt werden Phosphor-Ylide der Formel V, bei denen X ein Wasserstoffatom ist, während Y einen Phenyl-, Nitrophenyl-, Chlorphenyl-, Phenyl-$C_1$-$C_8$-alkoxy oder Furylrest darstellt.

Für die Durchführung der Wittig-Reaktion geht man zweckmäßigerweise von den entsprechenden Phosphoniumsalzen aus, die in einer Inertgasatmosphäre in einem inerten Lösungsmittel mit Hilfe einer geeigneten Base in die Phosphor-Ylide der Formel V umgewandelt werden.

Als Lösungsmittel eignen sich z. B. Dioxan, Tetrahydrofuran, Formamid, Dimethylformamid, Dimethylsulfoxid, Ethylenglykol-dimethylether, Diethylether, Cyclohexan, Benzol, Toluol, Xylol. Besonders bevorzugt wird Formamid oder ein Dioxan/Formamid-Gemisch, insbesondere im Verhältnis 95 : 5.

Als Basen finden z. V. Verwendung Natriumhydrid, Kaliumcarbonat, Natriumcarbonat, Kalium-tert.-butylat, Butyllithium, Phenyllithium. Speziell eignet sich Kaliumcarbonat in Verbindung mit Dioxan/Formamid.

Je nach Base und Lösungsmittel kann der Zusatz von Phasentransfer-Katalysatoren zweckmäßig sein, wie z. B. Kronenether, Tetrabutylammoniumhalogenide, KF/$Me_3$N·HCl oder KF/$R_3$N·HCl.

Die Reaktionstemperatur liegt üblicherweise zwischen -30° C und 180° C, insbesondere im Bereich von 60 bis 120° C und die Reaktion läuft bis zur vollständigen Umsetzung. Üblicherweise betragen die Reaktionszeiten 24 - 36 Stunden.

Die Darstellung der Phosphorane der Formel V und ihre Umsetzung mit Carbonylverbindungen sind in "Organic Reactions" Vol. 14, S. 270, J. Wiley and Sons; New York, London, Sidney, Toronto, 1965 beschrieben sowie in J. March: Advanced Organic Chemistry, Wiley Interscience,New York, Chichester, Brisbane, Toronto, Singapor 1985 Seite 845, sowie dort zitierte Literatur.

Vorzugsweise werden Verbindungen der Formel I oder II hergestellt, bei denen der Acyl-Rest durch einen $C_1$-bis $C_{18}$-Alkanoyl-, Benzoyl-, Trifluor-acetyl-, Benzyloxycarbonyl- oder t-Butyloxycarbonyl-Rest gebildet wird.

Bei der Umsetzung der D-Glucosaminderivate der Formel III oder IV mit Phosphor-Yliden der Formel V im Sinne einer Wittig-Reaktion erfolgt zunächst eine Epimerisierung, d. h. Inversion der Konfiguration am C2-Atom des Glucosaminderivats, woran sich die eigentliche Wittig-Olefinierung anschließt unter Bildung von Verbindungen der Formel VII bzw. VII.

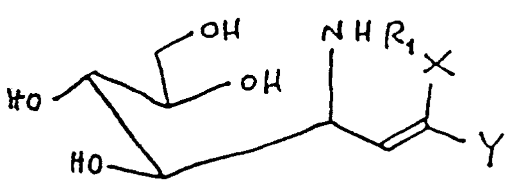

(VI)

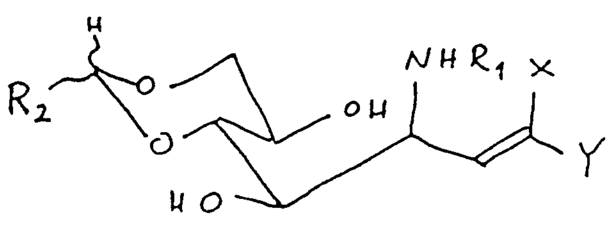

(VII)

Überraschenderweise wird durch diese Reaktion ein erheblicher Anteil an D-Mannosaminderivat erhalten und die säulenchromatographische Trennung der am Ende der Reaktion vorliegenden D-Mannosaminderivate von den D-Glucosaminderivaten wird relativ einfach erreicht. Im Falle von VI erfolgt die Reinigung noch einfacher durch Umkristallisieren.

Von den olefinischen Derivaten der Formeln VI bzw. VII gelangt man durch Oxidation mit Ozon zu den entsprechenden D-Mannosaminderivaten der Formeln I bzw. II. Durch geeignete Wahl der Temperatur kann bei der Ozonolyse der Verbindung der Formel VII direkt eine Verbindung der Formel I erhalten werden.

Die geschützten Derivate der Formel II, deren Rest $R_2$ vorzugsweise durch Phenyl, Methoxyphenyl oder Nitrophenyl gebildet wird, können als solche für weitere Synthesen verwendet oder auch durch katalytische Hydrierung mit Wasserstoff in die D-Mannosaminderivate der Formel I umgewandelt werden.

Nachfolgend wird die Erfindung anhand von Beispielen beschrieben.

Beispiel 1:

Herstellung von (E/Z)-3-Acetamido-1-phenyl-1,2,3-trideoxy-D-manno-hept-1-enitol und N-Acetyl-D-mannosamin aus N-Acetyl-D-glucosamin

Die Umsetzung erfolgt nach dem Schema:

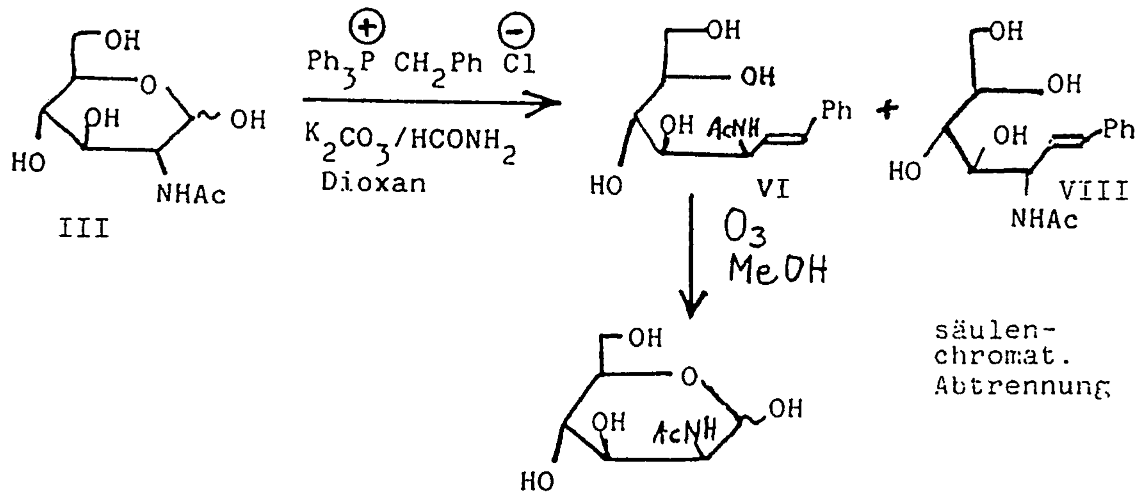


| Ansatz: | |
|---|---|
| N-Acetyl-D-glucosamin | 22,1 g ≙ 0,1 mol |
| Benzyltriphenylphosphoniumchlorid | 77,6 g ≙ 0,2 mol |
| Kaliumcarbonat | 27,6 g ≙ 0,2 mol |
| Formamid | ca. 20 ml |
| Dioxan | 500 ml |

Durchführung:

Glc-NAc, Phosphoniumsalz, $K_2CO_3$ und Dioxan wurden in einen 1l-Dreihalskolben mit Rührer und Rückflußkühler gegeben und der Luftsauerstoff mittels Argon aus der Apparatur entfernt. Nach Zugabe von ca. 20 ml Formamid wurde das Gemisch 24 - 36 h unter Rückfluß erhitzt. Nach Beendigung der Reaktion (Kontrolle durch Dünnschicht-Chromatographie mit Chloroform/Methanol 5 : 1) und Abkühlung wurden 50 ml Methanol zugesetzt und die Mischung 20 min lang gerührt. Die Lösung wurde abfiltriert, der Rückstand gründlich mit Dioxan gewaschen und das Filtrat zur Trockene eingedampft (unter Entfernung von Formamid im Hochvakuum).

Der erhaltene Feststoff wurde in 600 ml Wasser gelöst und die wäßrige Phase mit 400 ml Ether/Methylenchlorid-Mischung (3 : 1) dreimal gewaschen. Die organischen Phasen wurden zweimal mit je 100 ml Wasser rückextrahiert, die wäßrigen Phasen anschließend vereinigt und eingedampft. Säulenchromatographisch (Chloroform/Methanol 5 : 1) wurden dann 11,8 g der Verbindung VI und 5,9 g der Verbindung VIII erhalten. Gesamtausbeute ~60 %. Das Gemisch aus VI und VIII (2 : 1) hat einen Schmelzpunkt von 153 - 155 °C.

Ozonolyse:

2,95 g (10 mmol der reinen Verbindung VI wurden in 40 ml Methanol gelöst und so lange mit Ozon bei -60°C behan delt, bis das Edukt nicht mehr nachzuweisen war (DC, Chloroform/Methanol 5 : 1). Das überschüssige Ozon wurde mittels eines Stickstoffstroms ausgetrieben, anschließend wurde die Lösung mit 1-2 ml Dimethylsulfid versetzt. Nach 1 h bei Raumtemperatur wurden die flüchtigen Bestandteile in vakuo entfernt (zur Entfernung des entstandenen DMSO: Hochvakuum). Zur Entfernung des entstandenen Benzaldehyds wurde der Rückstand chromatographisch gereinigt (Chloroform/Methanol 5 : 1) . Alternativ wurde der Rückstand in wenig Wasser aufgenommen und mehrmals mit Ether ausgeschüttelt. Die wäßrige Phase wurde dann eingedampft.

Ausbeute an N-Acetyl-D-mannosamin x $1H_2O$: 2,27 g (~95 %).

Bespiel 2:

Herstellung von (E/Z)-3-Acetamido-5,7-O-benzyliden-1-phenyl-1,2,3-trideoxy-D-manno-hept-1-enitol

In einer zuvor ausgeheizten und mit Argon gespülten Apparatur wurden 80 ml Dioxan vorgelegt. Hierin wurden 5g 2-Acetamido-2-deoxy-4,6-O-benzyliden-D-glucose (17 mmol), 16,2 g Triphenyl-benzyl-phosphonium-chlorid (42 mmol) und 9,2 Kaliumcarbonat (66 mmol) suspendiert. Durch Zugabe von 2,7 ml Formamid (66 mmol) wurde die Reaktion gestartet. Die Reaktionsmischung wurde 10 h unter Rückfluß erhitzt. Der noch heißen Lösung wurden zur Vernichtung überschüssigen Ylids 2 ml Wasser zugesetzt. Nach Abziehen des Lösungsmittels verblieb ein gelblicher Sirup.

Die beiden Epimeren konnten chromatographisch ($CHCl_3$/MeOH 14 : 1) getrennt werden (Rf = 0,4 (VII) bzw. Rf = 0,3 (VIII-Derivat).

Alternativ wurde analysenreines Produkt (2,6 g) (VII) (10 %) durch Umkristallisieren des Rohsirups aus EE/EtOH/Ether erhalten (EE = Essigester) (E/Z = 7:1 F = 206°C). (Ausbeute nach Chromatographie:50 %;durch Umkristallisieren: 40 % ohne Aufarbeiten der Mutterlängen.)

$^1$H-NMR-Analyse (400MHz, DMSO)

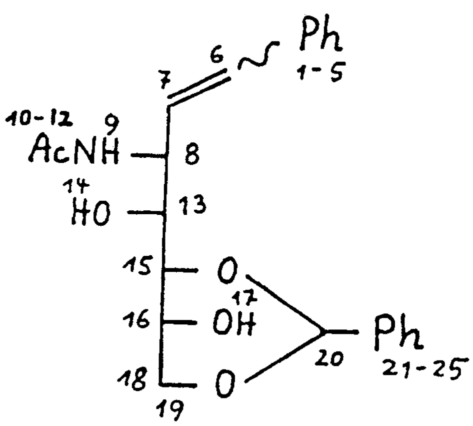

VII

= 1,80/1,90 ppm (s, 3H, (H-10-12), E/Z = 7:1),
3,50-5,20 ppm (m, 8H, (H-8,13-19))
5,45 ppm (s, 1H, (H-20))
5,65-5,75 ppm (dd,$J_{6,7}$ = 12 Hz, $J_{7,8}$ = 9,5 Hz, (H-7))
6,40-6,55 ppm (m, $J_{6,7}$ = 12 Hz, $J_{6,7}$ = 16 Hz, (H-6,7))
7,20-7,50 ppm (m, 10H, (H-1-5, 21-25))
7,85/7,95 ppm (d, 1H, (H-9),

| | | |
|---|---|---|
| HR-MS | Ber. für $C_{22}H_{25}NO_5$ ($M^+$): | 383, 1740 |
| | Gef. : | 383, 1736 (12 %) |

384 (1,3 %), 383 (12), 382 (4), 366 (2), 365 (10), 216 (20), 175 (80), 174 (80), 132 (100), 115 (50), 103 (40),

84 (25), 60 (12)

| Elementaranalyse | Ber.: | C = 68,91 %, | H = 6,57 %, | N = 3,65 % |
| --- | --- | --- | --- | --- |
| | Gef.: | C = 69,23 %, | H = 6,48 %, | N = 3,68 % |

383 mg ( 1 mmol ) der Verbindung VII (R = Acetyl) wurden wie in Beispiel 1 ozonolysiert. Danach wurde die Lösung eingedampft und der Rückstand mit einer katalytischen Menge Pt $0_2$ versetzt und 8 Stunden hydriert. Nach Filtration und Entfernung des Lösungsmittels wurden 220 mg ( 94 % ) N-Acetyl-D-Mannosamin Hydrat erhalten. Schmelzpunkt 110° - 112° C.

**Ansprüche**

1. Verfahren zur Herstellung von D-Mannosaminderivaten der Formel I oder II

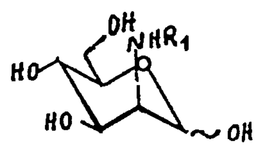

(I)

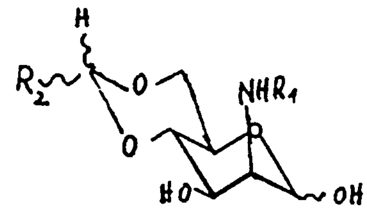

(II)

in der $R_1$ ein Acylrest und $R_2$ ein gegebenenfalls substituierter aromatischer Rest ist,
**dadurch gekennzeichnet,**
daß man das entsprechende D-Glucosaminderivat der Formel III oder IV

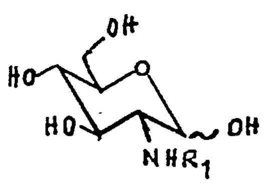

(III)

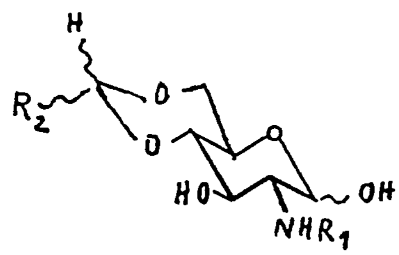

(IV)

in der $R_1$ und $R_2$ die bereits angegebene Bedeutung haben, in einer Wittig-Reaktion mit einem Phosphor-Ylid unter Epimerisierung umsetzt und das gebildete olefinische D-Mannosaminderivat nach Abtrennung des D-Glucosaminderivats mit Ozon oxydiert.

2. Verfahren nach Anspruch 1,
**dadurch gekennzeichnet,**
daß man eine so erhaltene Verbindung der Formel II mit Wasserstoff katalytisch zur entsprechenden Verbindung der Formel I hydriert.

3. Verfahren nach Anspruch 1,
**dadurch gekennzeichnet,**
daß man D-Mannosaminderivate aus den entsprechenden D-Glucosaminderivaten herstellt, bei denen $R_1$ ein $C_1$- bis $C_{18}$-Alkanoyl-, Benzoyl-, Trifluoracetyl-, Benzyloxycarbonyl- oder t-Butyloxycarbonyl-Rest ist.

4. Verfahren nach Anspruch 1,
**dadurch gekennzeichnet,**
daß man D-Mannosaminderivate der Formel II aus den entsprechenden D-Glucosaminderivaten herstellt, bei

denen $R_2$ ein Phenyl-, Methoxyphenyl- oder Nitrophenyl-Rest ist.

5. Verfahren nach einem der vorangehenden Ansprüche,

**dadurch gekennzeichnet,**

daß man als Phosphor-Ylid eine Verbindung der Formel V

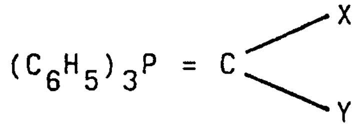

(V)

verwendet, in der X und Y gleich oder verschieden sein können und ein Wasserstoffatom, einen Phenyl-, Nitrophenyl-, Chlorphenyl-, Phenyl-$C_1$-$C_8$-alkoxy oder Furylrest bedeuten, wobei X und Y nicht gleichzeitig für Wasserstoff stehen sollen.

6. Verfahren nach einem der vorangehenden Ansprüche,

**dadurch gekennzeichnet,**

daß man die Wittig-Reaktion in einer Inertgasatmosphäre in einem inerten Lösungsmittel bei -30 °C bis 180 °C über 1 bis 36 Stunden hinweg ablaufen läßt.

7. Verfahren nach einem der vorangehenden Ansprüche,

**dadurch gekennzeichnet,**

daß man das durch Wittig-Reaktion gebildete olefinische D-Mannosaminderivat durch Säulenchromatographie mit Chloroform/Methanol (5 : 1) vom D-Glucosaminderivat abtrennt.

8. Verfahren nach einem der vorangehenden Ansprüche,

**dadurch gekennzeichnet,**

daß man durch Wittig-Reaktion gebildete olefinische D-Mannosaminderivate der Formel VII durch Umkristallisieren insbesondere aus Essigester/Ethanol/Ether vom D-Glucosaminderivat abtrennt.